# EUROPEAN PATENT APPLICATION

(11) **EP 1 400 587 A1**
(43) Date of publication of application: **24.03.2004**
(21) Application number: 02020941.7
(22) Date of filing: 19.09.2002
(51) Int. Cl.: C12N 5/06, A61K 35/12

(54) **Use of a fusion polypeptide for obtaining pluripotent non-embryonic stem cells**

(71) Applicant: MainGen Biotechnologie GmbH, 60314 Frankfurt/Main (DE)
(72) Inventor: Knaus, Rainer, Dr., 63773 Goldbach (DE); Schröder, Bernd, Dr., 17166 Teterow (DE); Rose-John, Stefan, Dr., 24211 Schellhorn (DE)
(74) Representative: Schüssler, Andrea, Dr.

(57) **Abstract**

Described is the use of a fusion polypeptide comprising (a) a ligand which is a member of the interleukin-6 (IL-6) family and (b) the receptor of said ligand, wherein - in a preferred embodiment - both parts (a) and (b) of said fusion polypeptide are linked by a polypeptide linker, or a vector capable of expressing said fusion polypeptide in secretable form, for obtaining and/or propagating a pluripotent non-embryonic stem cell.

## Description

The present invention relates to the use of a fusion polypeptide comprising (a) a ligand which is a member of the interleukin-6 (IL-6) family and (b) the receptor of said ligand, wherein, in a preferred embodiment, both parts (a) and (b) of said fusion polypeptide are linked by a polypeptide linker, or a vector capable of expressing said fusion polypeptide in secretable form, for obtaining and/or propagating a pluripotent non-embryonic stem cell.

Embryonic stem (ES) cells are the archetypical stem cells, being capable of differentiating to form the whole gamut of cell types found in the adult organism. Such stem cells are described as pluripotent cells as they are capable of differentiating into many cell types. Although ES cells have been isolated from human embryonic tissues, their use in research as well as therapeutics is encumbered by ethical considerations. Stem cells also exist for most tissues including haematopoietic, neural, gastrointestinal, epidermal, hepatic and mesenchymal stem cells. However, these stem cells have less self-renewal capacity and a more restricted capacity for differentiation, i.e. they are not pluripotent.

Until recently, it was thought that tissue-specific stem cells could only differentiate into cells of the tissue of origin. However, recent studies suggested that tissue-specific stem cells can differentiate into lineages other than the tissue of origin. After transplantation of bone marrow or enriched heamatopoietic stem cells, skeletal myoblasts, cardiac myoblasts, endothelium, hepatic and biliary duct epithelium, lung, gut and skin epithelia, and neuroectodermal cells of donor origin have been detected. Some studies demonstrated that neural stem cells as well as muscle cells may differentiate into haematopoietic cells. When injected into a blastocyst, neural stem cells contribute to a number of tissues of the chimeric mouse embryo. However, the studies carried out so far could not conclusively demonstrate that a tissue specific stem cell can differentiate into functional cells of multiple tissues, i.e. can be made pluripotent. To summarize, although there has developed a pressing need to obtain non-embryonic (human) pluripotent stem cells (adult stem cells), hitherto the problems associated with stable long term culture for the propagation of such stem cells including the problem of lack of pluripotency have not been solved.

Thus, the technical problem underlying the present invention is to provide means allowing to obtain non-embryonic stem cells that are pluripotent.

The solution to the above technical problem has been achieved by providing the embodiments characterized in the claims. It has surprisingly been found that when stem cells isolated from murine or human bone marrow are cultivated in the presence of a fusion polypeptide comprising IL-6 and a IL-6-receptor, in particular a fusion polypeptide ("Hyper-Il-6; H-IL-6) comprising an sIL-6R-polypeptide (extracellular or soluble domain of the interleukin-6 receptor) and an IL-6-polypeptide linked by a polypeptide linker, pluripotent stem cells can be obtained. These cells differentiate, at the single cell level, not only in mesenchymal cells, but also cells with visceral mesoderm, neuroderm and endoderm characteristics in vitro. It has been found that the fusion polypeptide activates gp130 which is a membrane protein of all cells. Furthermore, it has been found that this membrane protein is an activator of the protein STAT3 which governs the differentiation in embryonic stem cells.

Additionally it has been found that after injection of the pluripotent mouse stem cells into an early mouse blastocyst, these cells contribute to most, if not all, somatic cell types. On transplantation into a host, the cells engraft and differentiate to the haematopoietic lineage, in addition to the epithelium of liver, lung and gut. As these pluripotent mouse stem cells proliferate extensively without obvious senescence or loss of differentiation potential, i.e. they do not lead to the development of tumors as has been observed for embryonic stem cells, they are an ideal cell source for the treatment of various diseases, e.g. for the therapy of inherited or degenerative diseases.

Thus, the present invention relates to the use of a fusion polypeptide comprising (a) a ligand which is a member of the interleukin-6 (IL-6) family and (b) the receptor of said ligand, or a vector capable of expressing said fusion polypeptide in secretable form, for obtaining and/or propagating a pluripotent non-embryonic stem cell. The present invention also relates to a method for obtaining and/or propagating said stem cells comprising cultivating cells isolated from, e.g., bone marrow, cord blood, fat tissue, in the presence of said fusion polypeptide.

As used herein, the term "non-embryonic stem cell" comprises any non-embryonic stem cell, e.g. a gonadal stem cell or somatic stem/progenitor cell, such as haematopoietic stem cell, epidermal stem cell, mesenchymal stem cell or neuronal stem cell with a stem cell derived from the haematopoietic system being preferred. Sources for obtaining and/or propagating pluripotent versions of said stem cells according to the method of the present invention are e.g. bone marrow, peripheral blood, cord blood, fat tissue, liver, muscle, skeletal myoblasts, cardiac myoblasts, endothelium and epithelium.

As used herein, the term "fusion polypeptide comprising (a) a ligand which is a member of the interleukin 6 (IL-6) family and (b) the receptor of said ligand" refers to any fusion polypeptide comprising a ligand of the IL-6 family (or the biologically active part thereof) and its corresponding receptor (or part of the receptor which is responsible for binding of the ligand). The ligand and/or the receptor may comprise wild type sequences as well as sequences differing from the wild type sequences, e.g. by deletion(s), substitution(s) and/or addition(s) of amino acids. Such differences may result in ligands and/or receptors exhibiting improved or new biological activities, e.g., an improved binding of the ligand to its receptor.

The term "ligand which is a member of the interleukin 6 (IL-6) family" preferably relates to IL-6, IL-11, CNTF, OSM, LIF, CT-1 or CLC.

In a preferred embodiment, both parts (a) and (b) of said fusion protein are linked by a suitable polypeptide linker, e.g., ensuring that parts (a) and (b) can interact with each other. Suitable polypeptide linkers are known to the person skilled in the art and, e.g., described in the German Patent DE 196 08 813.

In a more preferred embodiment, the receptor of said fusion polypeptide is the ligand binding subunit and/or the ligand is the receptor binding subunit. The person skilled in the art can identify these domains of the ligand or receptor by standard methods, e.g., experimentally or by commonly available computer programs.

In an even more preferred embodiment, the ligand of said fusion polypeptide is human IL-6 and the receptor is the human IL-6 receptor with the soluble receptor (sIL-6R) being particularly preferred.

In the most preferred embodiment, said fusion protein is Hyper-IL-6 (IL-6/sIL-6R fusion polypeptide), i.e., a fusion polypeptide comprising a human sIL-6R polypeptide and a human IL-6 polypeptide, wherein both polypeptides are linked by a polypeptide linker. The primary structure of Hyper-IL-6 is described in DE 196 08 813 C2 and in Fischer et al., Nature Biotechnology, Vol. 15 (1997), 142-145. As used herein, the term "Hyper-IL-6" comprises (a) the fusion polypeptide having the amino acid sequence disclosed in DE 196 08 813 C2 and (b) a fusion protein having an amino acid sequence differing from the amino acid sequence of (a) with substantially the same biological activity.

The stem cells can be contacted with the fusion polypeptide by several methods, e.g. (a) culturing the cells in a medium containing said fusion polypeptide, (b) transfecting the cells with a vector intracellularly expressing said fusion protein in a secretable form, or (c) co-culturing the cells with producer cells expressing said fusion protein in a secretable form. The person skilled in the art knows suitable methods for transfection and suitable vectors; see e.g. DE 196 08 813 C2. The person skilled in the art can decide which approach is the most suitable one.

The person skilled in the art also knows suitable methods for culturing the stem cells and suitable media. The basal medium containing the fusion polypeptide of the invention can be any medium which is generally used for cultivation of mammalian (human) cells; see, e.g., DE 196 08 813 C2, EP-B1 0 695 351 and Example 1, below In a preferred embodiment, the basal medium containing the fusion polypeptide of the invention is IMEM (Life Technologies, Karlsruhe, Germany) or Cellgro (Cellgenix, Freiburg, Germany). The person skilled in the art can determine the optimum concentration of the fusion polypeptide by simple experimentation; see also DE 196 08 813 C2. Preferably, the concentration of the fusion polypeptide is in the range of 5 to 100 ng/ml medium.

The fusion polypeptide of the present invention used as additive to a medium can be prepared according to standard methods. Preferably, the fusion protein is recombinantly produced as, e.g., described in Example 2 of DE 196 08 813 C2 using the DNA sequence described in Example 1 of DE 196 08 813 C2.

The fusion protein may not be used directly, i.e. as an additive to a medium, but it can also be supplied to the stem cells by intracellular expression and subsequent secretion. For intracellular expression any vector capable of replicating in mammalian cells, preferably an expression vector, can be used. Preferably, the DNA sequence encoding the fusion protein is operatively linked to a suitable promoter. Suitable (expression) vectors are known to the person skilled in the art. Preferred recombinant vectors are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Furthermore, lenti viruses, such as HIV or SIV, are also preferred.

The construction of the recombinant vectors can be carried out according to conventional methods (cf. Sambrook et al., 1989, Molecular Cloning: A Laboratory Manual, 2^{nd} edition, Cold Spring Harbor Laboratory Press, NY, USA). These vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts for amplifying said vectors include, but are not limited to, bacterial cells, such as E. coli, Streptomyces and Salmonella typhimurium cells, fungal cells, such as yeast cells, insect cells such as Drosophila S2 and Spodoptera Sf9 cells, animal cells, and plant cells.

Introduction of the above described vectors into the stem cell can be effected by well known methods, e.g. calcium phosphate transfection, DEAE-dextran mediated transfection, cationic lipid-mediated transfection, electroporation, nucleofection, transduction, infection, or other methods. Such methods are described in many standard laboratory manuals.

The following Examples illustrate the invention.

### Example 1: Obtaining and propagating pluripotent human and murine stem cells

For obtaining and propagating pluripotent human and murine stem cells, the method described in Reyes et al., Blood 98 (2001), 2615-2625, is used for expansion.
**(A)** Human bone marrow mononuclear cells (BMMNCs), obtained by Ficoll-paque density gradient centrifugation are depleted of CD45+ and glycophorin A positive (GlyA+) cells by means of micromagnetic beats. The cells are plated on fibronectin, laminin and collagen type 4. The eluted cells are more than 95% CD45-/GlyA-cells. These cells are cultivated in a medium containing 0-10% fetal calf serum (FCS), 0-10ng/ml EGF and 0-10 ng/ml PDGF-BB as well as 5-100ng/ml Hyper IL-6. The cells are subcultured at 10 cells/well and maintained between 0,5-1,5 x 10³ cells/cm². Approximately 1% of wells seeded with 10 CD45-/GlyA-cells yield continuous growing pluripotent human stem cells.
**(B)** Murine bone marrow mononuclear cells (BMMNCs), obtained by Ficoll-paque density gradient centrifugation are plated on fibronectin, laminin, collagen type 4 and matrigel coated plates. The cells are cultivated in a medium containing 1-25ng/ml of EGF and PDGF-BB as well as 5-100ng/ml Hyper IL-6. After 3 weeks, remaining cells are selected by depletion of CD45+ and TER119 positive (TER119+) cells by means of micromagnetic beats. The cells are subcultured at 10 cells/well and maintained between 0,5-1,5 x 10³ cells/cm². Approximately 1% of wells seeded with 10 CD45-/TER110-cells yield continuous growing pluripotent murine stem cells.

### Example 2: In vitro differentiation of single pluripotent stem cells

About 1% of wells seeded with ten BMMNCs (of human and mouse origin, respectively) treated as described in Example 1 yield continuous growing cultures, suggesting that 1 out of 1,000 BMMNCs is capable of initiating pluripotent stem cell cultures and that progeny generated from ten cells is probably derived from one cell. To definitively prove that a single cell gives rise to continuous growing cultures and differentiated progeny, retroviral marking is used. For doing so, BMMNCs are transduced with an MoMuLV-eGFP retrovirus (Becker et al., Hum. Gene Ther. (1999), 1561-1570). After sub-cloning at ten cells per well, enhanced green fluorescent protein expressing (eGFP⁺) cell populations are selected and culture-expanded for more than 100 population doublings. It can be expected that all cells will continue to express GFP after expansion. Demonstration that progeny of only a single eGFP-transduced pluripotent stem cell gives rise to the continuous growing populations is achieved by Southern blot analyses and PCR using probes and primers specific for eGFP-DNA.

The in vitro differentiation ability of stem cells obtained according to the method described in Example 1 is tested by adding cytokines chosen on the basis of what has been described for differentiation of embryonic stem cells to mesoderm, neuroectoderm and endoderm. Differentiation requires that the stem cells are replated at 1-2 x 10⁴ cells cm⁻² in medium containing 60% DMEM-LG, 40% MCDB-201, ITS, LA-BSA, 10⁻⁹ M dexamethasone, 10⁻⁴ M ascorbic acid-2-phosphate, 100 U penicillin and 1.000 Units streptomycin (Reyes et al., Blood 98 (2001) , 2615-2625; Reyes et al., J. Clin. Invest. 109 (2002), 337-346) but without serum and Hyper-IL-6. Differentiation is induced by lineage-specific cytokines. After culturing, cells are fixed with 4% paraformaldehyde and methanol at room temperature and incubated sequentially for 30 min each with primary and secondary antibodies. Between steps, slides are washed with PBS/BSA. Cells are examined by confocal fluorescence microscopy. To assess the frequency of different cell types in a given culture, the number of cells staining positive with a given antibody are counted in four low-lower visual fields; 200 to 500 cells per field.

As an example of mesoderm differentiation to endothelium is induced by culturing the pluripotent stem cells on fibronectin (FN) with 10 ng/ml vascular endothelial growth factor (VEGF)-B for 14 days (Reyes et al., Blood 98 (2001), 2615-2625; Reyes et al., J. Clin. Invest. 109 (2002), 337-346). Most of the pluripotent stem cells aquire an endothelial phenotype and express CD31, Flk-1 and vWF. All cells staining positive for vWF also express eGFP.

For neuroectodermal differentiation, 1 x 10⁴ cells per cm² are plated on FN with 100 ng/ml bFGF (R&D Systems). Alternatively, cells are treated sequentially with 100 ng/ml bFGF for 7 days, 10 ng/ml PGF-8 for 7 days and 10 ng/ml BDNF for 7 days (R&D Systems) (Palmer et al., J. Neurosci. 19 (1999), 8487-8497). After 14 days, some of the pluripotent stem cells acquire morphologic and phenotypic characteristics of astrocytes (glial acidic fibrillary protein (GFAP)⁺), some of oligodendrocytes (galactocerebroside (GalC)⁺) and most of them of neurons (neurofilament-200 (NF-200)⁺). NF-200, GFAP or GalC are not found in the same cell. Quantitative RT-PCR of pluripotent stem cells treated with bFGF confirms expression of neuroectodermal genes. The following types of cells can be found: cells expressing markers (a) of dopamine-containing neurons (dopa-decarboxlyse (DDC) and tyrosine hydroxylase (TH), (b) of scrotonin-containing (serotonin positive) neurons and (c) of γ-aminobutyric acid (GABA)-containing (GABA positive) neurons. Neuron-like cells become polarized, as Tau and MAP2 are expressed in axonal and somatodentritic compartments, respectively.

Next, differentiation to endodermal cells is tested. As described by Schwartz et al., J. Clin. Invest. 109 (2002), 1291-1302, when replated on matrigel with 10 ng/ml FGF-4 and 20 ng/ml hepatocyte growth factor (HGF), most of the pluripotent stem cells aquire epithelioid morphology and some cells become binucleated. Most cells stain positive for albumin, cytokeratin (CK)18 and HNF-1 with all cells staining positive for CK18 being also eGFP⁺. Southern blot analyses and flanking region PCR demonstrate that differentiated eGFP⁺ pluripotent stem cells contain the identical viral insertion site found in undifferentiated stem cells. Since these experiments show that all differentiated cells are eGFP⁺ and that only a single viral insertion site is present, it is apparent that a single pluripotent stem cell differentiates into cells with morphologic, phenotypic and functional characteristics of cells representing the three germ layers.

### Example 3: Further determination of the extent of differentiation of the pluripotent stem cells.

To further determine the extent of differentiation of the pluripotent stem cells from murine bone marrow obtained according the approach of Example 1, the ability to contribute to various tissues is assessed by introducing the mouse pluripotent stem cells into an early blastocyst. One or 10 to 12 cells obtained after 55 to 65 population doublings are microinjected into 3.5-day old blastocysts of C57BL/6 mice. Blastocysts are transferred to foster mothers and mice are allowed to develop and be born. Animals born from microinjected blasocysts are of similar size as normal animals and do not display overt abnormalities.

Tissue collection and analysis: For the whole-mouse mount, 10-µm whole body sections near the midline are prepared as described (Reinhardt et al., Nature 401 (2001), 101-105). Tissue sections are stained for β-galactosidase enzyme activity with the β-gal staining kit from Invitrogen at pH 7.4 according to the manufacturer's instructions. A total of 0.5 to 1 ml of blood is obtained when animals are killed, and bone marrow is collected by flushing femurs and tibias. For phenotyping, red blood cells and bone marrow are depleted using ice-cold ammonium chloride (Stem Cell Technologies Inc.) and 10⁵ cells are used for cytospin centrifugation.

Chimaerism is assessed by comparing levels of Neo/β-Gal (Zambrowicz et al., PNAS USA 94 (1997), 3789-3794) in tail dippings of 4-week old animals with that of tissue of mice used as the source for the pluripotent stem cells of Example 1 using Q-PCR. Chimaerism can be detected in most of mice derived from blastocysts in which 10 to 12 stem cells are injected and in many mice derived from blastocysts microinjected with one pluripotent stem cell. Chimearism ranges between 0.1% and 45%. Absence of chimaerism in some of the microinjected blastocysts may indicate that the pluripotent mouse stem cells are not completely homogeneous.

Animals are killed at 6 to 20 weeks. Some mice are frozen in liquid nitrogen, thin whole-mouse sections cut as described by Reinhardt et al., Nature 401 (2001), 101-105, and stained with 5-bromo-4-chloro-3-indolyl-β-D-galactoside (X-Gal). Blue X-Gal staining indicates chimaerism.

Then, multiple organs are collected separately and the presence of cells derived from the pluripotent haematopoietic stem cells is determined by X-Gal staining and staining with an anti-β-gal-fluorescein isothiocyanate (FITC) antibody. Chimaeric animals containing Neo/β-Gal⁺ cells (as determined by PCR in the tail clip analysis) have a contribution of cells derived from the pluripotent haematopoietic stem cells to many tissues, including brain, retina, lung, myocardium, skletelal muscle, liver, intestine, kidney, spleen, bone marrow, blood and skin (as shown by X-gal staining). B-gal⁺ cells express markers typical for the tissue in which they have incorporated. β-gal⁺ cells in bone marrow, spleen and blood co-stain for CD45, Gr-1, Mac-1, CD19 and CD3 antigens. Because of the haematopoietic chimaerism, triple-colour immunofluorescence is used to assure that β-gal⁺ cells in solid organs are not mere haematopoietic cells, β-gal⁺ co-stains for pan-CK in the lung and intestine, and for CK18 in the liver. No cell co-stains for all three antigens (i.e. β-gal, CD45 and CK). β-gal⁺ cell co-stain for dystrophin in skeletal muscle and cardiac troponin-1 in the myocardium. It has been found that β-gal⁺ cells will give rise to neurons (Neu-N⁺) and astrocytes (GFAP⁺) throughout the entire brain, including the cortex, striatum, hippocampus, thalamus and cerebellum. To conclude, a single pluripotent non-embryonic stem cell generates balanced chimaeras, i.e., contributes to most somatic cell types, and chimaerism can be detected not only in mouse embryos, but also in mice that are 6 to 20 weeks old.

### Example 4: Pluripotent non-embryonic mouse stem cells infused intravenously engraft and differentiate in tissue-specific cells

It was tested whether the pluripotent non-embryonic mouse stem cells when infused intravenously in post-natal animals engraft and differentiate in tissue-specific cells. To avoid rejection by recipient animals, the cells are injected by means of the tail vein into non-irradiated or irradiated (250 cGy) 4 to 8 week-old non-obese diabetic/severe combined immunodeficient (NOD/SCID) recipients. For study of engraftment, lungs are inflated with 1 ml 1:4 dilution of optimum cutting temperature (OCT) compound (Sakuar-Finetek Inc.) in PBS. Specimens of spleen, liver, lung, intestine, skeletal muscle, myocardium, kidney and brain of the recipient animals are collected and cryopreserved in OCT at -80°C and in RNA zol (Rot, Germany) at - 20°C for Q-PCR. Five-micrometer-thick fresh frozen sections are mounted on glass slides and fixed in acetone for 10 min at room temperature. After incubation with isotype sera for 20 min, slides are stained sequentially with antibodies against cell-type antigens, anti-β-gal antibody, in some instances with antibodies against CD45, or a nuclear counterstain (4,6-diamidino-2-phenylindole (DAPI) or TO-PRO-3).

Engraftment, defined as more than 1% β-gal⁺ cells by immunofluorescence and/or Q-PCR, is seen in haematopoietic tissues (blood, bone marrow and spleen) and epithelium of lung, liver and intestine of all recipient animals, and is similar in animals analysed 4 to 24 weeks after transplantation.

β-gal⁺ cells in bone marrow and spleen co-stain for CD45. Most of bone marrow β-gal⁺ co-stain for Gr-1 and many of the cells co-stain for Mac-1, for CD19 and for TER119. Similar results are seen for blood cells. No β-gal⁺ CD3⁺ T cells are seen in blood, bone marrow or spleen even though β-gal⁺ CD3⁺ cells are present in chimaeric mice. Engraftment in the spleen occures mostly as clusters of donor cells, consistent with the hypothesis that when the stem cells home to the spleen, they proliferate locally and differentiate to form a colony of donor cells, similar to colony-forming unit spleen (CFU-S). Engraftment of the pluripotent non-embryonic mouse stem cells is also seen in liver, intestine and lung. Because of the haematopoietic engraftment, triple-colour immunohistochemistry is used to discriminate between epithelial and haematopoietic cells in the same tissue sections.

In contrast with ES cells, donor-derived tumors can not be detected in any of the immunodeficient mice that received the pluripotent non-embryonic mouse stem cells intravenously.

## Claims

1. Use of a fusion polypeptide comprising (a) a ligand which is a member of the interleukin 6 (IL-6) family and (b) the receptor of said ligand, or a vector capable of expressing said fusion polypeptide in secretable form, for obtaining and/or propagating a pluripotent non-embryonic stem cell.

2. Use according to claim 1, wherein both parts of the fusion polypeptide are linked by a polypeptide linker.

3. Use according to claim 1 or 2, wherein the receptor of said fusion polypeptide is the ligand binding subunit and/or the ligand is the receptor binding subunit.

4. Use according to any one of claims 1 to 3, wherein the ligand is IL-6 and the receptor is the IL-6 receptor.

5. Use according to claim 4, wherein the fusion polypeptide is Hyper-IL-6.

6. Use according to any one of claims 1 to 5, wherein said stem cell is a gonadal stem cell or somatic stem/progenitor cell.

7. Use according to claim 6, wherein said stem cell is a haematopoietic stem cell, epidermal stem cell or neuronal stem cell.

8. Use according to any one of claims 1 to 7, wherein said stem cell is a human stem cell.

9. Use according to any one of claims 1 to 8, wherein said fusion protein is present in a culture medium in a concentration between 5 and 100 ng/ml.
